# EUROPEAN PATENT APPLICATION

(11) **EP 3 228 345 A1**
(43) Date of publication of application: **11.10.2017**
(21) Application number: 16163666.7
(22) Date of filing: 04.04.2016
(51) Int. Cl.: A61M 11/00, A24F 47/00, A61M 15/06

(54) **INHALER AND LIQUID COMPOSITION**

(71) Applicant: Nexvap SA, 1070 Puidoux (CH)
(72) Inventor: Lacour-Gayet, Julien, 1090 Lacroix-sur-Lutry (CH)
(74) Representative: Hepp Wenger Ryffel AG

(57) **Abstract**

An inhaler with an elongated housing in the shape of an e-cigarette includes a mouthpiece (1), a body element (2) detachably connected to the mouthpiece (1), a nebulizer (3) and a container (4) for a liquid containing nicotine or other soluble chemical compounds. The container is connected or connectable to the nebulizer (3) in a fluid connection. The the nebulizer (3) is a vibrating mesh type nebulizer.

## Description

The invention relates to an inhaler with a mesh type nebulizer in the shape of an e-cigarette, a liquid composition for an inhaler, a kit comprising an inhaler and a liquid composition (conveying nicotine or other soluble or miscible chemical compounds), a method for administering nicotine and/or other chemical compounds and a container cartridge for an inhaler device according to the independent claims.

Cigarette smoking is linked to the combustion of tobacco and other compounds, which generate up to 5000 different combustion products and are known to be the cause of different diseases including chronical obstructive pulmonary diseases (COPD), cancer and cardiovascular diseases. It is the combustion that creates harmful and potentially harmful chemical compounds.

A new type of nicotine consumption with so-called e-cigarettes or Electronic Nicotine Delivery System (ENDS) has emerged in the last decade.

In an e-cigarette or ENDS in general, a liquid composition containing nicotine, propylene glycol and vegetable glycerine (and other miscible chemical components) is evaporated by means of the heating of a coil surrounding a mesh. The mesh is a fabric that pumps the liquid by capillarity (usually cotton). When the coil surrounding the mesh (an electrical resistance) is placed under electric tension, the mesh heats to temperatures from 80 degrees Celsius to 300 degrees Celsius, which results in the vaporization of the liquid contained in the mesh. The user then inhales the vapour.

Although an e-cigarette or ENDS has recently been recognized as being a safer alternative to a normal cigarette (e.g. Public Health England recognising that e-cigarettes are 95% less risky than combustible tobacco), there are still different disadvantages linked with the use of e-cigarettes.

Due to the vaporization of the liquid composition by heat as well as due to the composition of the liquid, traces of toxic or potentially toxic compounds can still be produced in an e-cigarette through various heat-induced chemical reactions. This is the case even if the original liquid composition does not contain any harmful components: it is the heating that produces the toxic components. To a lesser extent, some materials used in an e-cigarette (metals and plastics) may generate additional harmful chemical compounds when the temperature is elevated.

Other known types of e-cigarettes use an ultrasonic device to create the vapour (in this case, an aerosol), avoiding heating and the production of potentially harmful chemical components. However, these ultrasound based e-cigarettes produce an aerosol with a droplet size too large to penetrate deeply into the lung. Such e-cigarettes are therefore not fully suited for a nicotine replacement therapy usage (NRT) under medical and pharmaceutical standards.

It is an aim of the present invention to overcome the drawbacks of the prior art and in particular to provide a device and a composition for nicotine replacement therapy which do not lead to generation of toxic or potentially toxic compounds and which nevertheless will generate droplets with a size suitable for penetrating deeply into the lung.

This aim is solved by an inhaler according to the independent claim.

The inhaler comprises an elongated housing in the shape of an e-cigarette including a mouthpiece, a body element detachably connected to the mouthpiece, a nebulizer and a container for a liquid containing nicotine or other miscible chemical compounds suitable for Nicotine Replacement Therapy, where nicotine is partly or fully replaced by other chemical compounds (such as any miscible pharmaceutical drug or nutrient, such as e.g. Aspirin, CAS Number 88566-80-7). The container is connected or connectable to the nebulizer by a fluid connection. The nebulizer is a vibrating mesh type nebulizer. Replacing the traditional heat-based coil by the vibration of a mesh not only suppresses all heat induced chemical reactions in the liquid, but also delivers an aerosol with measurable, small and constant droplet size, which in turn allows targeted delivery deep in the lung. This is particularly suited for the use in a pharmaceutically approved Nicotine Replacement Therapy.

The shape of an e-cigarette is typically a substantially cylindrical shape having a diameter from 12mm to 14 mm and a length of 140 mm to 180mm.

Nebulizers are normally used for the treatment of cystic fibrosis, asthma and other respiratory diseases and are bulk shaped. Their likely acceptance for the use as an NRT is therefore low. An e-cigarette shape is likely to be better accepted by a user, since its use is more associated to smoking.

The inhaler comprises an essentially cylindrically shaped housing in the form of an e-cigarette. The housing comprises a mouthpiece for the inhalation of the vapour. The mouthpiece is detachably connected to a body element of the housing, which normally contains a battery, a battery charging port (e.g. mi-cro-USB), and an electronic control system. A container for the liquid containing nicotine or other miscible chemical compounds is further provided and is arranged within the housing in fluid connection with a nebulizer. The container may be a separated container or may be an integral part of the housing, in particular of the mouthpiece. The nebulizer is a vibrating mesh type nebulizer known as such. Such a vibrating mesh type nebulizer comprises a vibrating plate with a plurality of preferably tapered openings. The tapering is in flow direction of the liquid composition. That means that the opening cross section where droplets are generated is smaller than the opening cross section where the liquid composition is provided. The vibrating plate is operatively connected to a vibration generator, in particular a piezo-electric vibration generator. Preferably, the vibrating plate is surrounded by a vibration generator.

The vibration generator and an electronic control are preferably adapted to vibrate the plate with a frequency of up to 100'000 movements per second.

The plate is preferably provided with more than 100 openings per square millimetre, more preferred with more than 500 or more than 2000 openings. Typically there may be 1'000 to 10'000 openings, more preferably about 2'000 to 6'000 opening and most preferably about 4'000 openings per square millimetre. The tapered openings act as a micro-pump and generate droplets of the liquid containing nicotine or other soluble chemical compounds. The droplets are then inhaled by the user.

Preferably the vibrating plate is dome shaped.

Preferably, the vibrating plate has a thickness between 2 and 4 mm. The openings preferably have a diameter of 400 to 600 nm, preferably about 500 nm on the inside and 800 to 1'200 nm, preferably about 1 micron on the outside of the plate.

With an inhaler according to the invention, there is no heat vaporization of the liquid composition. Therefore, there is also no heat-induced modification of the components of the liquid containing nicotine or other soluble chemical compounds, with no formation of toxic or potentially toxic compounds.

The inhaler may further comprise a communication interface for exchanging data with another electronic device. The communication interface may be wireless (e.g. WLAN, Bluetooth). It is also possible to use a charging port (e.g. micro-USB) for cable bound transmission of data. The data is preferably related to the liquid and therefore to the nicotine (or other chemical compounds) consumption of a user. The communication interface may also be used for programming the electronic control unit of the inhaler in a NRT such that, e.g., an user may only inhale e predefined amount of nicotine during a given time interval. Such an interface also allows monitoring patient compliance with a therapy regime.

The inhaler is further preferably designed with the container being detachable from the housing. The container is filled with a liquid containing nicotine or other miscible chemical compounds, preferably as described hereinabove, and sealed with a perforable membrane. Upon insertion of the container into the housing, preferably into the mouthpiece, and connection of the mouthpiece with the body element, the membrane is perforated or teared by a member of the nebulizer, thus providing a fluid connection between the container and the nebulizer. Such a container is particularly useful since there is no need for handling with bottles of liquid that may lead to leaking.

The container preferably has a central channel for the vapour, which, upon insertion of the container into the housing, is connected to the opening of the mouthpiece and allows passage of the vapour generated by the nebulizer.

The aim of the invention is further solved with a liquid composition according to the independent claim.

The invention also relates to a liquid containing nicotine or other soluble chemical compounds usable in the inhaler described above, that includes 1,3-Propanediol and nicotine and/or other soluble chemical compound suitable for nicotine replacement therapy.

The advantage of such a composition, in particular when used in combination with vibrating mesh inhalers, is that there is no formation of potentially toxic compounds such as acrolein, acetaldehyde and formaldehyde, which can be generated in conventional e-cigarettes when a liquid composition containing propylene glycol and vegetable glycerine is vaporized. Furthermore, a composition according to the invention allows an optimal and stable delivery of nicotine and/or other soluble chemical compounds suitable for nicotine replacement therapy in the gas phase.

The composition preferably has a content of 1,3-Propanediol between 10% w/w and 66% w/w.

The composition may further include at least one aroma, preferably food grade aroma(s).

The aroma content of the composition is preferably not higher than 2% w/w. Since 1,3-Propanediol is a flavour enhancer, a lower aroma concentration than in conventional nicotine solution is possible. Therefore high aroma concentrations (higher than the authorized concentrations in foods) with unknown toxicity and consequences when inhaled can be avoided.

The composition preferably further contains a surfactant in order to reduce the surface tension of the droplets in the aerosol phase. Preferably at least one surfactant is selected from the following surfactant list: anionic surfactants, non-ionic surfactants, cationic surfactants, amphoteric or zwitterionic surfactants, polymeric surfactants or surface active polymers. The surfactant has the advantage of stabilizing the surface tension of the liposomes or micelles created in the aerosol phase.

The surfactant content of the composition is preferably not higher than 1% w/w.

Preferably the composition further contains a solution-based electrolyte, preferably NaCl. The electrolyte's presence in the liquid prevents droplets in the aerosol phase to merge into larger droplets. Alternatively, other electrolytes than NaCl may be used.

Typically a physiological NaCl solution is used, with Sodium Chloride in an amount of 0.9% w/v, where Na+ is 3.54 g/l (154 mmol/l) and Cl- 5.46 g/l (154 mmol/l), with an osmolarity of 308 mosm/l.

The physiological solution preferably is sterile.

The content of electrolyte solution in the composition is preferably between 30% w/w and 84% w/w.

The composition preferably further contains a viscosity enhancer, e.g. fructose. The viscosity enhancer increases the density of the liquid, in particular to counterbalance the volumetric weight of certain aromas.

The viscosity enhancer content is preferably not higher than 1% w/w.

The composition does not contain propylene glycol.

Furthermore, by balancing the content of the 1,3-Propanediol with other components, in particular the viscosity enhancer and the electrolyte, compositions with different characteristics and properties can be provided. In particular it is possible to decrease the vapour that is exhaled by a user, thus making the composition suitable for use in enclosed spaces (offices, public transport, restaurants, airplanes, etc.) where normal smoking is usually prohibited.

Preferably the inhaler according to the invention is used with a liquid containing nicotine or other soluble chemical compounds according to the present invention. The inhaler may be used with liquids that do not contain nicotine, where nicotine is replaced by other chemical compounds.

The invention also relates to a kit comprising an inhaler and a liquid containing nicotine or other soluble chemical compounds for inhalation.

The inhaler of the kit comprises a mesh type nebulizer and is in particular an inhaler according to the present invention.

The liquid of the kit is also preferably the solution according to the present invention.

A further aspect of the present invention is to provide a method for administering nicotine or other soluble chemical compounds to a user.

The method according to the present invention includes the step of generating an aerosol of a liquid containing nicotine or other soluble chemical compounds suitable for nicotine replacement therapy by means of a mesh type nebulizer. The mesh type nebulizer is preferably a nebulizer as described above.

The invention is described below in more detail by means of preferred embodiments in connection with the drawing. It is shown in:

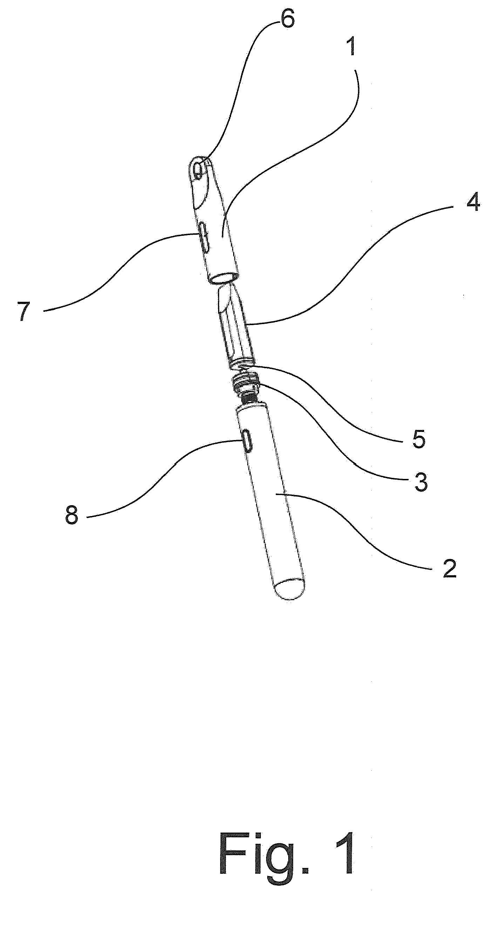

- Fig. 1: an exploded view of an inhaler according to the present invention;

Figure 1 shows an inhaler according to the present invention. The inhaler comprises a mouthpiece 1 and a body element 2 which, when joined together form a housing of the inhaler in the shape of an e-cigarette. The mouthpiece 1 and body element 2 are screwed together.

A nebulizer 3 is arranged inside the housing. A liquid container 4 is arranged inside the mouthpiece 1 and is connected in a fluid connection with the nebulizer 3. The container 4 is arranged within the mouthpiece 4. The container 4 has a central through hole 5 that allows the passage of vapour from the nebulizer 3 to a opening 6 of the mouthpiece 1.

The container 4 is filled with a liquid containing nicotine or other soluble chemical compounds and is made of a transparent plastic material. The mouthpiece 1 is provided with a side view opening 7 which allows a user to optically check the level of liquid in the container 4.

A button 8 is used for operating the nebulizer and is pushed by a user when inhaling in order to produce vapour.

The housing is circular cylindrical with a diameter of 14 mm and a length of 180mm.

The body contains further elements such as an electronic control, a battery or a battery charging port (e.g. micro-USB) and a wireless data transmission interface. These components are known as such and are not shown in the drawing.

The nebulizer 3 is a vibrating mesh type nebulizer known as such (e.g. such as Omron nebulizers for deep lung deposition). The nebulizer 3 comprises a dome shaped vibrating plate with a plurality of tapered openings and with a thickness of about 3mm. The vibrating plate is operatively connected to a piezo-electric vibration generator (not shown), vibrating the plate with a frequency of up to 100'000 Hz.

The plate has 4'000 openings per square millimetre, on a total surface of 2 square millimetres.

The openings preferably have a diameter of 500 nm on the inside and 1 micron on the outside of the plate.

The composition used in the inhaler is described below. If not stated differently, all content indications are in w/w percent.

A first example is a mixture which shows a high visible vapour generation when exhaled. The mixture comprises 66% 1,3-Propanediol, 2% nicotine and 2% of a food grade aroma. The remainder of the volume is a physiological sodium chloride solution.

A second example is a mixture showing less vapour generation than the mixture of the first example. The mixture comprises between 45% and up to 50% 1,3-Propanediol, 2% nicotine, 1% of a surfactant, 1% of a viscosity enhancer and 2% of a food grade aroma. The the remainder of the volume is a physiological sodium chloride solution.

A third example is a mixture showing practically no vapour generation compared to the mixtures described above. The mixture comprises 10% 1,3-Propanediol, 2% nicotine, 1% of a surfactant, between 1% and up to 5% of a viscosity enhancer and 2% of a food grade aroma. The remainder of the volume is a physiological sodium chloride solution.

Specific embodiments of the three examples are shown in the tables below:

**Example 1:**

| | |
|---|---|
| 1,3-Propanediol | 66% |
| Nicotine | 2% |
| Zingerone, CAS Number 122-48-5 (Aroma) | 2% |
| Physiological NaCl solution | 30% |

**Example 2:**

| | |
|---|---|
| 1,3-Propanediol | 50% |
| Nicotine | 2% |
| Poractant Alfa, CAS Number 129069-19-8 (Surfactant) | 1% |
| Zingerone, CAS Number 122-48-5 (Aroma) | 2% |
| Glycerin, CAS Number 8043-29-6, (Viscosity enhancer) | 1% |
| Physiological NaCl solution | 44% |

**Example 3:**

| | |
|---|---|
| 1,3-Propanediol | 10% |
| Nicotine | 1% |
| Poractant Alfa, CAS Number 129069-19-8 (Surfactant) | 1% |
| Zingerone, CAS Number 122-48-5 (Aroma) | 2% |
| Glycerin, CAS Number 8043-29-6, (Viscosity enhancer) | 3% |
| Physiological NaCl solution | 83% |

## Claims

1. An inhaler with an elongated housing in the shape of an e-cigarette, including a mouthpiece (1), a body element (2) detachably connected to the mouthpiece (1), a nebulizer (3) and a container (4) for a liquid containing nicotine or other soluble chemical compounds, which container is connected or connectable to the nebulizer (3) thereby forming a fluid connection, wherein the nebulizer (3) is a vibrating mesh type nebulizer.

2. Inhaler according to claim 1, wherein the nebulizer (3) comprises a dome shaped vibrating plate, in particular a piezo-electrically operated vibrating plate.

3. Inhaler according to claim 2, wherein the vibrating plate is adopted to be vibrated with a frequency of up to 100'000 upward and downward movements per second.

4. Inhaler according to one of the claims 1 to 3, wherein the vibrating plate has a thickness between 2 and 4 mm and/or wherein the openings have a diameter of 400 to 600 nm, preferably about 500 nm on the inside and 800 to 1'200 nm, preferably about 1 micron on the outside of the plate.

5. Inhaler according to one of the claims 1 to 4, wherein the vibrating plate has more than 100 openings per square millimetre, preferably more than 500, most preferably more than 2000 openings per square millimetre.

6. Inhaler according to claim 5, wherein the vibrating plate has 1'000 to 10'000 openings, preferably about 2'000 to 6'000 openings and most preferably about 4'000 openings per square millimetre.

7. Inhaler, in particular according to one of the claims 1 to 6, further comprising an electronic control unit and a communication interface for communication of data stored in the electronic control unit to a remote electronic device.

8. A liquid composition for an inhaler, in particular for an inhaler according to one of the claim 1 to 7, including 1,3-Propanediol and at least one of nicotine or another compound suitable for nicotine replacement therapy.

9. The composition according to claim 8, wherein the content of 1,3-Propanediol is between 10% w/w and 66% w/w.

10. The composition according to one of the claims 8 or 9, further containing at least one aroma, the aroma content preferably being not higher than 2% w/w.

11. The composition according to one of the claims 8 to 10, further containing a surfactant, preferably selected from the group of anionic surfactants, non-ionic surfactants, cationic surfactants, amphoteric or zwitterionic surfactants, polymeric surfactants or surface active polymers, the surfactant content preferably being not higher than 1% w/w.

12. The composition according to one of the claims 8 to 11, further containing an electrolyte solution, preferably a NaCl solution, the amount of the electrolyte solution content preferably being between 30% w/w and 84% w/w.

13. The composition according to claim 12, wherein the electrolyte is provided as a physiological solution

14. The composition according to one of the claims 8 to 13, further containing a viscosity enhancer, preferably fructose,
wherein the viscosity enhancer content preferably is not higher than 1% w/w.

15. The composition according to one of the claims 8 to 14,
wherein the composition does not contain propylene glycol.

16. Kit comprising a vibrating mesh type nebulizer, preferably an inhaler according to one of the claims 1 to 7 and a liquid containing nicotine or other soluble chemical compounds, preferably a composition according to one of the claims 8 to 15.

17. Method of administering nicotine or other soluble chemical compound suitable for nicotine replacement therapy, comprising the step of generating an aerosol of a liquid containing nicotine or other soluble chemical compounds, in particular a composition according to one of the claims 8 to 16, by means of a mesh type nebulizer.

18. Container (4) for a liquid composition for a inhaler, in particular containing a composition according to one of the claim 8 to 15, comprising a body defining a filling cavity and a membrane sealing the filling cavity, wherein the membrane is perforable by a member of an inhaler upon insertion of the container into the housing of said inhaler, thus providing a fluid connection between the container and the nebulizer.
